# EUROPÄISCHE PATENTSCHRIFT

(11) **EP 4 262 937 B1**
(45) Veröffentlichungstag und Bekanntmachung des Hinweises auf die Patenterteilung: **20.05.2026**
(21) Anmeldenummer: 21823782.4
(22) Anmeldetag: 25.11.2021
(51) Int. Cl.: A61M 5/32

(54) **INJEKTIONSVORRICHTUNG MIT EINER KAPPE ZUR ENTFERNUNG EINER NADELSCHUTZKAPPE VON EINEM PRODUKTBEHÄLTER UND VERFAHREN ZUM VORBEREITEN EINER SOLCHEN VORRICHTUNG**
INJECTION DEVICE WITH A CAP FOR REMOVING A NEEDLE PROTECTION CAP FROM A PRODUCT CONTAINER, AND METHOD FOR PREPARING SUCH A DEVICE
DISPOSITIF D'INJECTION AVEC UN CAPUCHON POUR RETIRER UN CAPUCHON DE PROTECTION D'AIGUILLE D'UN RÉCIPIENT DE PRODUIT, ET MÉTHODE DE PRÉPARATION D'UN TEL DISPOSITIF

(30) Priorität: 17.12.2020 CH 16052020
(43) Veröffentlichungstag der Anmeldung: 25.10.2023
(73) Patentinhaber: Ypsomed AG, 3401 Burgdorf (CH)
(72) Erfinder: URBANEK, Leos, 3012 Bern (CH); HIRSCHEL, Jürg, 3007 Bern (CH); ALLENSPACH, Marcel, 3400 Burgdorf (CH)
(74) Vertreter: Eugster, Monika Katharina
(86) Internationale Anmeldenummer: PCT/EP2021/082945
(87) Internationale Veröffentlichungsnummer: WO 2022/128385

(56) Entgegenhaltungen:
- US-A1- 2013 274 655
- US-A1- 2015 051 553
- US-A1- 2019 001 070

## Beschreibung

Die Erfindung betrifft eine Injektionsvorrichtung zur Verabreichung eines flüssigen Produkts, insbesondere eines Medikaments.

Der Begriff "Medikament" umfasst hier jede fliessfähige medizinische Formulierung, welche geeignet ist zur kontrollierten Verabreichung durch ein Mittel wie eine Kanüle oder Injektionsnadel hindurch, beispielsweise umfassend eine Flüssigkeit, eine Lösung, ein Gel, oder eine feine Suspension, welche einen oder mehrere medizinische Wirkstoffe enthält. Medikament kann eine Zusammensetzung mit einem einzigen Wirkstoff oder eine vorgemischte oder co-formulierte Zusammensetzung mit mehreren Wirkstoffen aus einem einzelnen Behälter sein. Medikament umfasst Arzneien wie Peptide (z.B. Insuline, Insulin enthaltende Medikamente, GLP-1 enthaltende sowie abgeleitete oder analoge Zubereitungen), Proteine und Hormone, biologisch gewonnene oder aktive Wirkstoffe, Wirkstoffe auf Basis von Hormonen oder Genen, Nährformulierungen, Enzymen und weitere Substanzen sowohl in fester (suspendierter) oder flüssiger Form aber auch Polysaccaride, Vaccine, DNS oder RNS oder Oglionukleotide, Antikörper oder Teile von Antikörpern sowie geeignete Basis-, Hilfs- und Trägerstoffe.

Aus dem Stand der Technik sind Injektionsvorrichtungen bekannt, in denen eine Fertigspritze angeordnet ist. Die Fertigspritze weist eine Injektionsnadel auf, die unlösbar mit der Fertigspritze verbunden ist und über die ein in der Fertigspritze enthaltenes Medikament ausgegeben werden kann. Um die Injektionsnadel und das Medikament der Fertigspritze steril zu halten, wird die Injektionsnadel von einer an der Fertigspritze befestigten Nadelschutzkappe umschlossen und in Bezug auf die Umgebung steril abgedichtet. Solche Nadelschutzkappen können z.B. als sogenannte soft needle shield (SNS) oder als rigid needle shield (RNS) ausgestaltet sein. Ein rigid needle shield (RNS) weist mehrere Teile auf, insbesondere ein elastomeres kappenförmiges Teil und ein aus einem festen, d.h. nicht-elastomeren Kunststoff hergestellten hülsenförmiges Teil, welches das elastomere Teil aufnimmt und damit im Wesentlichen unlösbar verbunden ist.

Um ein in der Fertigspritze enthaltenes Medikament injizieren zu können, muss die Nadelschutzkappe von der Fertigspritze entfernt werden. Aus der WO2010/136076 A1, der US 9,339,610 B2, der WO 2015/144871 A1 und US 2016/0243315 A1 ist bekannt, dass beim Abziehen eines kappenförmigen Abziehelements, das auch als Kappe bezeichnet wird, am distalen Ende der Injektionsvorrichtung angebracht ist und das distale Ende der Injektionsvorrichtung verschliesst, die an der Fertigspritze angebrachte Nadelschutzkappe mitabgezogen, d.h. beim Entfernen der Kappe von der Fertigspritze entfernt wird. Die Nadelschutzkappe verbleibt dabei in der Kappe. Hierzu weist die Kappe Eingriffsglieder auf, die beim Abziehen der Kappe in Eingriff mit der Nadelschutzkappe gebracht werden. Beim Fortsetzen der Abziehbewegung des Abziehelements nehmen die Eingriffsglieder die Nadelschutzkappe mit, wodurch diese von der Fertigspritze abgezogen wird. Um ein sicheres Abziehen der Nadelschutzkappe durch das Entfernen der Kappe zu gewährleisten, ist es aus dem Stand der Technik bekannt, dass die mit der Kappe verbundenen Eingriffsglieder in Eingriff mit der Nadelschutzkappe gelangen. Ferner ist aus der EP 2255842 B1 bekannt, dass die Nadelschutzkappe durch Drehung der Kappe von der Fertigspritze abgezogen werden kann. Weitere Beispiele aus dem Stand der Technik sind aus der US2019/001070, der US2015/051553, und der US2013/274655 bekannt.

Es ist eine Aufgabe der Erfindung, eine Injektionsvorrichtung und ein Verfahren zum Vorbereiten einer solchen Injektionsvorrichtung für die Verabreichung eines Produkts anzugeben, die ein einfacheres Entfernen der Nadelschutzkappe von dem Produktbehälter erlaubt.

Die Aufgabe wird mit der Injektionsvorrichtung nach Anspruch 1 gelöst Vorteilhafte Weiterbildungen ergeben sich aus den abhängigen Ansprüchen, der Beschreibung und den Figuren.

Die Erfindung geht von einer Vorrichtung zur Verabreichung eines Produkts, nämlich von einer Injektionsvorrichtung mit einer Längsachse (L), aus. Die Injektionsvorrichtung kann als so genannter Autoinjektor ausgestaltet sein, der einen Mechanismus aufweist, der ein automatisches Ausschütten des Produkts, wie z. B. durch ein Antriebselement, insbesondere eine Feder, und optional ein automatisches Einstechen und/oder Zurückziehen der Injektionsnadel bewirkt. Bei einem Autoinjektor wird die Kraft zum Ausschütten des Produkts durch das Antriebselement, wie z.B. die Feder bereitgestellt. Die Injektionsvorrichtung kann alternativ als manuelle Injektionsvorrichtung ausgestaltet sein, d.h., dass die Kraft für die Ausschüttung des Produkts durch Muskelkraft, wie z.B. durch den Benutzer selbst erfolgt. Die Injektionsvorrichtung - egal ob es sich um einen Autoinjektor oder eine manuelle Injektionsvorrichtung handelt - kann eine Nadelschutzhülse aufweisen, die nach erfolgter Injektion distal über das distale Ende der Injektionsnadel steht oder relativ zu einem Gehäuse der Injektionsvorrichtung in diese Position verschoben wird, um den versehentlichen Zugriff auf die Injektionsnadel zu verhindern und dadurch ein Verletzungsrisiko zu verringern. Optional kann die Nadelschutzhülse auch schon vor der Injektion distal über das distale Ende der Injektionsnadel hinaus stehen. Bei einem Autoinjektor kann die Nadelschutzhülse beispielsweise auch als Auslöseelement zum Auslösen der Produktausschüttung dienen, wobei die hierzu relativ zu dem Gehäuse der Injektionsvorrichtung in die proximale Richtung verschoben wird. Alternativ kann die Auslösung des Autoinjektors durch Betätigen eines Auslöseknopfs des Autoinjektors erreicht werden, wobei die Nadelschutzhülse vor dem Gebrauch des Autoinjektors als Sichtschutz dient.

Die Injektionsvorrichtung weist einen Produktbehälter mit einer Injektionsnadel auf, wie z. B. eine aus dem Stand der Technik bekannte Fertigspritze oder allgemein Spritze. Der Produktbehälter kann einen z.B. hohlzylindrischen Produktbehälterabschnitt aufweisen, der einen Kolben verschiebbar lagert. Der Kolben kann mit dem Innenumfang des Produktbehälterabschnitts eine Dichtung bilden und so eine sterile Barriere bilden. Der Kolben kann z. B. mittels einer Kolbenstange der Injektionsvorrichtung in die distale Richtung verschoben werden, um über die Injektionsnadel Produkt aus dem Produktbehälter abzugeben. Die Injektionsnadel kann vorzugsweise unlösbar an dem Produktbehälter gebildet sein. Zum Beispiel kann der Produktbehälter einen Halteabschnitt, insbesondere einen Nadelhalteabschnitt, aufweisen, der distal des Produktbehälterabschnitts angeordnet ist und mit der Injektionsnadel unlösbar verbunden ist, und so zum Beispiel einen proximalen Teil der Injektionsnadel umgibt. Die Injektionsnadel kann somit von dem Halteabschnitt in die distale Richtung abragen. Der Halteabschnitt kann beispielsweise einen geringeren Aussendurchmesser als der Produktbehälterabschnitt aufweisen. Der Produktbehälterabschnitt kann sich an seinem distalen Ende zu dem Halteabschnitt hin verjüngen.

Der hierin verwendete Begriff "distal" bezieht sich auf die Richtung, in die die Spitze der Injektionsnadel zeigt. Der hierin verwendete Begriff "proximal" bezieht sich auf die Richtung, die der distalen Richtung entgegengesetzt ist.

An dem Produktbehälter, beispielsweise an dem Halteabschnitt, ist eine Nadelschutzkappe, wie z. B. ein aus dem Stand der Technik bekanntes soft needle shield (SNS) oder rigid needle shield (RNS), befestigt, insbesondere lösbar befestigt. Die Nadelschutzkappe kann z. B. reib- oder formschlüssig oder kombiniert reib- und formschlüssig auf dem Halteabschnitt befestigt sein. Die Nadelschutzkappe umschliesst die Injektionsnadel und dichtet sie in Bezug auf die Umgebung steril ab. Ein soft needle shield (SNS) umfasst ein oder besteht aus einem Elastomer, beispielsweise auf Gummi- oder Kautschukbasis gebildeten Teil, welches die Nadel umgibt. Das soft needle shield (SNS) weist an seinem Aussenumfang eine weiche, wie z. B. aus einem gummi- oder kautschukartigen Material gebildete Oberfläche auf. Ein rigid needle shield (RNS) weist zumeist mehrere Teile auf, insbesondere ein elastomeres kappenförmiges inneres Teil und ein aus einem steiferen, d. h. typischerweise nicht-elastomeren Kunststoff hergestelltes hülsenförmiges oder kappenförmiges äusseres Teil, welches das elastomere Teil aufnimmt und damit im Wesentlichen unlösbar verbunden ist. Das äussere hülsen- oder kappenförmige Teil umgibt das innere kappenförmige Teil und ist mit der inneren Kappe beispielsweise unlösbar verbunden, so dass die äussere und innere Kappe eine Einheit bilden. Das äussere Teil kann aus einem härteren Kunststoff als das innere Teil gebildet sein. Das äussere Teil kann beispielsweise aus Polyethylen, Polystyrol, Polypropylen oder einem anderen geeigneten Kunststoff sein. Das innere Teil kann beispielsweise aus Gummi oder Kautschuk oder einem anderen geeigneten Material gebildet sein.

An dem distalen Ende der Injektionsvorrichtung oder des Gehäuses, wie z. B. eines Aufnahmegehäuses der Injektionsvorrichtung kann eine Kappe, die auch als Verschlusskappe oder Abziehkappe bezeichnet werden oder ausgestaltet sein kann, befestigt sein und das distale Ende des Gehäuses oder des Aufnahmegehäuses verschliessen. Die Kappe kann einteilig oder mehrteilig ausgebildet sein. Die Injektionsvorrichtung kann ein Gehäuse, wie z.B. ein Aufnahmegehäuse der Injektionsvorrichtung zur Aufnahme des Produktbehälters umfassen, wobei der Produktbehälter eine fest verbundene Injektionsnadel aufweist und wobei an dem Produktbehälter die Nadelschutzkappe lösbar angeordnet ist. Die Nadelschutzkappe umschliesst die Injektionsnadel und dichtet die Injektionsnadel gegenüber der Umgebung steril ab. Die Kappe kann z. B. mit dem Gehäuse oder Aufnahmegehäuse und/oder mit der Nadelschutzhülse reib- und/oder formschlüssig verbunden sein, wie z. B. verschnappt sein. Die Kappe kann vorzugsweise aus Kunststoff gebildet sein. Alternativ kann die Kappe aus Metall gebildet sein. Die Kappe kann z. B. während des Entfernens von der Injektionsvorrichtung und/oder dem Gehäuse und/oder der Nadelschutzhülse mit einer kombinierten/seriellen Axial-Dreh-Bewegung oder einer Axialbewegung von der Injektionsvorrichtung, wie z. B. dem Gehäuse oder Aufnahmegehäuse und/oder Nadelschutzhülse, abnehmbar sein.

Die Injektionsvorrichtung kann ferner einen Produktbehälterhalter umfassen, welcher mit dem Gehäuse der Injektionsvorrichtung fest, insbesondere axial- und drehfest verbunden ist. Der Produktbehälterhalter kann zur Aufnahme des Produktbehälters dienen, wobei in dem Produktbehälterhalter der Produktbehälter fest, insbesondere axial- und vorzugsweise drehfest gehalten werden kann. Alternativ können das Gehäuse und der Produktbehälterhalter einteilig ausgebildet sein. Alternativ kann der Produktbehälterhalter relativ zu dem Gehäuse axial bewegbar und/oder drehbar angeordnet sein.

Die Kappe, welche lösbar an dem distalen Ende des Gehäuses oder am distalen Ende der Nadelschutzhülse der Injektionsvorrichtung vorgesehen ist, umfasst ein oder mehrere Eingriffselemente, um beim Entfernen der Kappe von der Injektionsvorrichtung das Entfernen der Nadelschutzkappe von dem Produktbehälter zu bewirken. Die Kappe, welche mit dem Eingriffselement gekoppelt ist, kann über das Eingriffselement derart mit der Nadelschutzkappe verbindbar sein, dass das Entfernen der Kappe von der Injektionsvorrichtung das Entfernen der Nadelschutzkappe von dem Produktbehälter bewirkt. Insbesondere kann zumindest ein Teil der Bewegung oder die gesamte Bewegung der Kappe in die distale Richtung auf das Eingriffselement übertragen werden, d. h., dass das Eingriffselement von der Kappe mitgenommen wird, so dass das Eingriffselement die Nadelschutzkappe von dem Produktbehälter, insbesondere dem Halteabschnitt, abzieht. Die Kappe und/oder das Eingriffselement können relativ zu dem Gehäuse und/oder relativ zu der Nadelschutzhülse drehbar angeordnet sein. Das Eingriffselement ist vorzugsweise axial- und drehfest mit der Kappe verbunden. Die Kappe und das Eingriffselement können einteilig oder mehrteilig ausgebildet sein.

In einer Ausführungsform kann das Eingriffselement derart verformbar sein, dass das Eingriffselement von einer beabstandeten Position, in welcher das Eingriffselement von der Nadelschutzkappe radial beabstandet ist, in eine Eingriffsposition, in welcher das Eingriffselement in Eingriff mit der Nadelschutzkappe ist, bringbar ist, wobei das Eingriffselement beim Entfernen der Kappe verformt wird. Das Eingriffselement kann z. B. im Auslieferungszustand der Injektionsvorrichtung in Bezug auf die Nadelschutzkappe in der beabstandeten Position sein. In der Eingriffsposition ist das Eingriffselement in Bezug auf die Nadelschutzkappe derart angeordnet, dass eine Bewegung der Kappe in die distale Richtung eine Mitnahme der Nadelschutzkappe bewirkt und somit die Nadelschutzkappe von dem Produktbehälter entfernt wird. In der Eingriffsposition des Eingriffselements greift das Eingriffselement an oder in die Nadelschutzkappe. Das Eingriffselement kann an oder in eine Mantelfläche oder an oder in eine Kante oder an oder in eine distalen Stirnfläche oder an oder in eine proximale Stirnfläche der Nadelschutzkappe greifen. Das Eingriffselement kann einen Haken oder mehrere Haken umfassen. Besonders bevorzugt kann das Eingriffselement zumindest teilweise hakenförmig ausgebildet sein. Besonders bevorzugt kann das Eingriffselement ferner hülsenförmig oder zylinderförmig ausgebildet sein. Das hakenförmige Eingriffselement kann einen kurzen und einen langen Schenkel aufweisen. Vorzugsweise kann der lange Schenkel verformbar ausgebildet sein. Ferner kann der kurze Schenkel zahn- oder dreieckförmig oder spitzwinklig ausgebildet sein. Alternativ kann das Eingriffselement eine andere Ausgestaltung aufweisen, wobei in der beabstandeten Position des Eingriffselements das Eingriffselement von der Nadelschutzkappe radial beabstandet ist und in der Eingriffsposition des Eingriffselements das Eingriffselement in Eingriff mit der Nadelschutzkappe ist, wobei das Eingriffselement beim oder vor dem Entfernen der Kappe verformt wird.

In der beabstandeten Position des Eingriffselements kann das Eingriffselement unverformt, verformt oder radial nach aussen verformt sein. In der Eingriffsposition des Eingriffselements kann das Eingriffselement unverformt, verformt oder radial nach innen verformt sein. Das Eingriffselement kann vorzugsweise plastisch und/oder elastisch verformbar sein.

Als eine plastische Verformung wird eine bleibende Verformung bezeichnet. Die Verformung eines Materials ist plastisch, wenn das Material nicht wieder von allein seine ursprüngliche Form annimmt. Nach Einwirkung von einer Kraft oder Belastung auf das Material behält das Material seine Form bei. Als eine elastische Verformung wird eine reversible Verformung bezeichnet. Dabei nimmt ein Material nach Einwirkung einer Kraft oder einer Belastung auf das Material wieder seine ursprüngliche Form an.

Das Eingriffselement ist vorzugsweise aus Metall, insbesondere aus Stahl, besonders bevorzugt aus rostfreiem Stahl oder Federstahl gebildet. Das Eingriffselement ist aus einem Material gebildet, welches eine Biegefestigkeit aufweist, welche eine plastische und/oder elastische Verformung zulässt. Besonders bevorzugt ist das Eingriffselement derart ausgebildet, dass es in der beabstandeten Position des Eingriffselements plastisch und/oder elastisch verformt und in der Eingriffsposition plastisch und/oder elastisch unverformt ausgebildet ist oder, dass es in der beabstandeten Position des Eingriffselements plastisch und/oder elastisch unverformt und in der Eingriffsposition plastisch und/oder elastisch verformt ausgebildet ist.

Ferner kann an dem Gehäuse oder an einem Gehäuse fest verbundenen Teil ein oder mehrere Sperrelemente vorgesehen sein, wobei in der Eingriffsposition das oder die Sperrelemente das Eingriffselement in Eingriff mit der Nadelschutzkappe hält oder bringt. Das Sperrelement kann eine erste und/oder eine zweite schräge Fläche, insbesondere eine erste und/oder eine zweite nach innen ragende schräge Fläche, umfassen. Die erste und/oder die zweite schräge Fläche des Sperrelements können eine Neigung aufweisen. Die erste und die zweite schräge Fläche können zueinander geneigt sein. Das Eingriffselement der Kappe kann derart mit dem Sperrelement des Gehäuses gekoppelt sein, dass während dem Entfernen der Kappe von der Injektionsvorrichtung das Eingriffselement relativ zu der Nadelschutzkappe bewegbar ist oder bewegt wird und bei dieser Bewegung, insbesondere einer kombinierten/seriellen Axial-Drehbewegung oder einer Axialbewegung, mittels dem Sperrelement des Gehäuses derart verformbar ist oder verformt wird, dass das Eingriffselement, insbesondere der kurze Schenkel des Eingriffselements in Eingriff mit der Nadelschutzkappe gelangbar ist oder gelangt. In der Eingriffsposition des Eingriffselements ist das Eingriffselement mit der Nadelschutzkappe axialfest verbunden, wobei die Nadelschutzkappe von dem Eingriffselement der Kappe bei der Fortführung der kombinierten/seriellen Axial-Drehbewegung oder der Axialbewegung der Kappe mitgenommen wird. Mit anderen Worten umfasst der Hub, den die Kappe beim Entfernen von der Injektionsvorrichtung relativ dem Gehäuse entlang der Längsachse (L) in die distale Richtung ausführt, einen ersten Teilhub, während dem die Kappe relativ zu der Nadelschutzkappe bewegbar ist oder bewegt wird, und einen zweiten Teilhub, während dem die Nadelschutzkappe die Bewegung der Kappe mitmacht oder von der Kappe mitgenommen wird.

In einer alternativen Ausführungsform kann ein oder mehrere Eingriffselemente vorgesehen sein, welche elastisch und/oder plastisch verformbar ausgebildet sind, wobei das oder die mehreren Eingriffselemente immer und/oder bereits beim Montieren einer Injektionsvorrichtung für die Verabreichung eines Produkts in einem Kontakt mit der Nadelschutzkappe sind. Dieses Montageverfahren umfasst ferner das Verschieben oder Einsetzen eines Produktbehälters mit der lösbar verbundenen Nadelschutzkappe in ein Gehäuse entlang einer Längsachse (L) in die distale Richtung, wobei das Gehäuse an einem distalen Ende eine Kappe aufweist. Eine Mantelaussenfläche der Nadelschutzkappe gleitet dabei axial über das oder die Eingriffselemente, insbesondere den oder die kurzen Schenkel des Eingriffselements. In der Position, in welcher der Produktbehälter in dem Gehäuse eingesetzt ist, sind das oder die Eingriffselemente der Kappe in einer Eingriffsposition. In dem Auslieferungszustand der Injektionsvorrichtung können somit das oder die Eingriffselemente der Kappe in einer Eingriffsposition sein.

In einer alternativen Ausführungsform kann die Kappe mit einem oder mit mehreren Eingriffselementen relativ zu einer Nadelschutzkappe in die proximale Richtung bewegt werden, um die Kappe auf ein Produktbehälter aufgenommenes Gehäuse aufzusetzen.

Die Mantelfläche der Nadelschutzkappe kann eine oder mehrere Öffnungen oder ein oder mehrere Befestigungsmittel aufweisen, in welche das Eingriffselement in der Eingriffsposition des Eingriffselements eingreifen oder sich einbohren kann. Alternativ weist die Nadelschutzkappe keine Öffnung oder kein Befestigungsmittel auf, wobei das Eingriffselement in der Eingriffsposition des Eingriffselements in die Mantelfläche der Nadelschutzkappe eingreifen oder sich einbohren kann.

Ferner kann die Injektionsvorrichtung ein Energiespeicherelement umfassen. Das Energiespeicherelement kann durch ein oder mehrere Halteelement in der Kappe gehalten werden, sodass das Energiespeicherelement nicht aus der Kappe fällt.

Als ein zumindest teilweises Entfernen der Nadelschutzkappe von dem Produktbehälter wird eine Kombination aus manuellem Entfernen, d.h. dass die Kraft für das Entfernen durch Muskelkraft, wie z.B. durch den Benutzer selbst erfolgt und aus automatischem Entfernen, d.h. dass die Kraft für das Entfernen durch das Energiespeicherelement, wie z.B. durch die Feder oder durch das Magnet erfolgt, bezeichnet. Dabei kann das Energiespeicherelement das manuelle Entfernen der Nadelschutzkappe von dem Produktbehälter unterstützen.

Das Energiespeicherelement kann in die distale Richtung an der Kappe und in die proximale Richtung an dem Gehäuse oder an der Nadelschutzhülse abgestützt sein.

Die Kappe kann relativ zu dem Gehäuse oder/und zu der Nadelschutzhülse drehbar sein. Diese relative Drehung kann zum Entfernen der Kappe von der Injektionsvorrichtung dienen. Dabei kann durch die relative Drehbewegung die Kappe entfernt werden oder ermöglichen, dass die Kappe entfernt werden kann.

Ferner kann an dem Gehäuse der Injektionsvorrichtung oder an der Nadelschutzhülse ein Element vorgesehen sein, welches in Führungseingriff mit einem an der Kappe vorgesehenen Gegenelement ist. Das Element und/oder das Gegenelement können als eine Führungskulisse/Führungsnut oder eine Führungsnocke ausgebildet sein. Besonders bevorzugt kann die Führungskulisse/Führungsnut ein Gewinde umfassen oder gewindeförmig ausgebildet sein. Alternativ kann die Führungskulisse/Führungsnut einen Absatz oder eine Treppe umfassen oder treppenförmig oder L-förmig ausgebildet sein.

Die Führungskulisse/Führungsnut kann vorzugsweise einen Absatz oder eine Treppe umfassen. Der Absatz oder die Treppe kann dazu dienen, dass das Element bzw. Gegenelement während der Führung durch den Absatz oder die Treppe gebremst wird. Somit kann ein unkontrolliertes Entfernen der Kappe von der Injektionsvorrichtung verhindert werden.

Besonders bevorzugt kann das Element axial- und drehfest mit dem Gehäuse oder der Nadelschutzhülse verbunden sein. Ferner kann das Gegenelement axial- und drehfest mit der Kappe verbunden sein. Ferner kann das Element mit dem Gehäuse oder der Nadelschutzhülse einteilig oder mehrteilig ausgebildet sein. Des Weiteren kann das Gegenelement mit der Kappe einteilig oder mehrteilig ausgebildet sein.

Die Kappe kann relativ zu dem Gehäuse oder relativ zu der Nadelschutzhülse drehbar sein, um die Kappe von dem Injektionsgerät zu entfernen. Bevorzugt können das Element und das Gegenelement relativ zu einander drehbar sein. Das Element und das Gegenelement können dazu ausgelegt sein, in einer ersten relativen Drehposition um die Längsachsel (L) zwischen dem Element und dem Gegenelement ein Entfernen der Kappe von der Injektionsvorrichtung zu verhindern und in einer zweiten relativen Drehposition um die Längsachsel (L) zwischen dem Element und dem Gegenelement ein Entfernen der Kappe von der Injektionsvorrichtung zu ermöglichen. Somit kann die Kappe kontrolliert bzw. nicht unbeabsichtigt, insbesondere mit Unterstützung des Energiespeicherelements, insbesondere mit Unterstützung der Kraft des Energiespeicherelements von der Injektionsvorrichtung entfernt werden.

Besonders bevorzugt können das Element oder das Gegenelement als Abstützelement des Energiespeicherelements in die proximale Richtung dienen. Somit kann die Injektionsvorrichtung kompakt ausgebildet werden.

Das Element und/oder das Gegenelement können vorzugsweise aus Kunststoff gebildet sein. Alternativ kann das Element und/oder Gegenelement aus Metall gebildet sein.

Erfindungsgemäß weist das Element oder das Gehäuse oder die Nadelschutzhülse ein Rückdrehsicherungselement und das Gegenelement oder die Kappe ein Rückdrehsicherungsgegenelement auf, um eine Drehung der Kappe um die Längsachse (L) in eine Richtung zu ermöglichen und in Gegenrichtung zu sperren. Der Rückdrehmechanismus der Injektionsvorrichtung dient dazu, dass der Benutzer die Kappe nur in eine Drehrichtung relativ zu dem Eingriffselement oder relativ zu dem Gehäuse oder relativ zu der Nadelschutzhülse drehen kann. Eine Drehung der Kappe um die Längsachse (L) ist in eine Richtung möglich und in die Gegenrichtung blockiert.

Besonders bevorzugt kann das Rückdrehsicherungselement und/oder das Rückdrehsicherungsgegenelement eine Verzahnung oder eine Verrastung aufweisen oder als Verzahnung oder als Verrastung ausgebildet sein. Dazu kann das Rückdrehsicherungselement ein oder mehrere Zähne aufweisen, welche mit einem oder mit mehreren Zähnen des Rückdrehsicherungsgegenelements derart zusammenwirken, dass eine Drehung der Kappe um die Längsachse (L) in eine Richtung möglich ist und in Gegenrichtung gesperrt ist. Die korrespondierende Verzahnung oder Verrastung umfassen entsprechende Gleitflächen und Anschlagsflächen auf, um eine Drehung der Kappe um die Längsachse (L) in eine Richtung zu ermöglichen und in Gegenrichtung zu sperren.

In einer alternativen Ausführungsform kann das Rückdrehsicherungselement und/oder das Rückdrehsicherungsgegenelement ein Federarm aufweisen oder als Federarm ausgebildet sein. Ferner kann das Rückdrehsicherungselement und/oder das Rückdrehsicherungsgegenelement einen Vorsprung, Absatz, Vertiefung, Rille oder Aussparung aufweisen, welche um die Längsachse (L) in eine Richtung möglich ist und in Gegenrichtung gesperrt ist.

Alternative Ausgestaltungen des Rückdrehsicherungselements und/oder des Rückdrehsicherungsgegenelements können vorgesehen sein, soweit dass eine Drehung um die Längsachse (L) in eine Richtung möglich ist und in Gegenrichtung gesperrt wird.

Ferner kann die Injektionsvorrichtung, insbesondere die Kappe, das Eingriffselement und/oder das Gehäuse eine visuelle Markungen, insbesondere in Form eines Zeichens, besonders bevorzugt in Form eines Pfeils aufweisen, um anzuzeigen in welche Richtung eine relative Drehung der Kappe zu dem Gehäuse oder zu der Nadelschutzhülse um die Längsachse (L) möglich ist. Diese Markungen dient der einfacheren Bedienung der Injektionsvorrichtung. In alternativen Ausführungsformen kann zusätzlich oder alternativ eine akustische und/oder taktile Markungen vorgesehen sein.

Besonders bevorzugt ist die Nadelschutzhülse an oder in der Injektionsvorrichtung vorgesehen, wobei die Kappe über die Nadelschutzhülse mit dem Gehäuse lösbar verbunden ist. Die Nadelschutzhülse ist vorzugsweise relativ zu dem Gehäuse drehfest angeordnet. Die Nadelschutzhülse dient dazu, vor oder nach erfolgter Injektion distal über das distale Ende der Injektionsnadel zu stehen. Die Nadelschutzhülse ist teilweise von dem Gehäuse aufgenommen, wobei an einem distalen Ende der Nadelschutzhülse die Kappe aufgesetzt werden kann. Die Kappe kann z.B. mit der Nadelschutzhülse reib- und/oder formschlüssig verbunden sein, wie z.B. verschnappt werden. Alternativ kann die Kappe mit dem Gehäuse lösbar verbunden sein, wobei die Injektionsvorrichtung eine oder keine Nadelschutzhülse umfassen kann.

Ergänzend wird auf die Merkmale, die im Zusammenhang mit der hierin beschriebenen Vorrichtung offenbart werden, verwiesen, die auch das Verfahren vorteilhaft weiterbildet.
- Figur 1: eine Explosionsansicht eines distalen Endes einer ersten Ausführungsform einer erfindungsgemässen Injektionsvorrichtung mit einer Längsachse (L).
- Figur 2: eine Längsschnittansicht der Injektionsvorrichtung gemäss Figur 1, wobei die Kappe (1) relativ zu der Nadelschutzhülse (3) in einer ersten relativen Drehposition angeordnet ist.
- Figur 3: eine Perspektivenansicht der Figur 2, wobei ein Teil der Kappe (1) ausgespart ist.
- Figur 4: eine Perspektivenansicht der Injektionsvorrichtung gemäss Figur 1, wobei ein Teil der Kappe (1) ausgespart ist und wobei die Kappe (1) relativ zu der Nadelschutzhülse (3) in einer zweiten relativen Drehposition angeordnet ist.
- Figur 5: eine Längsschnittansicht der Kappe (1) gemäss Figur 1, wobei die Kappe (1) von der Injektionsvorrichtung abgenommen ist und die Nadelschutzkappe (5a) hält.
- Figur 6: eine Explosionsansicht eines distalen Endes einer zweiten Ausführungsform einer erfindungsgemässen Injektionsvorrichtung mit einer Längsachse (L).
- Figur 7: eine Längsschnittansicht der Injektionsvorrichtung gemäss Figur 6, wobei die Kappe (1c, 1d) relativ zu dem Gehäuse (4') in einer ersten relativen Drehposition angeordnet ist.
- Figur 8: eine Längsschnittansicht der Kappe (1c, 1d) gemäss Figur 6, wobei die Kappe (1c, 1d) von der Injektionsvorrichtung abgenommen ist und die Nadelschutzkappe (5a) hält.
- Figur 9: eine Perspektivenansicht der Figur 8, wobei die Nadelschutzkappe (5a') und die Feder nicht gezeigt ist.
- Figur 10: eine Längsschnittansicht der Injektionsvorrichtung gemäss Figur 6, wobei die Kappe (1c, 1d) von der Injektionsvorrichtung abgenommen ist und somit nicht ersichtlich ist.

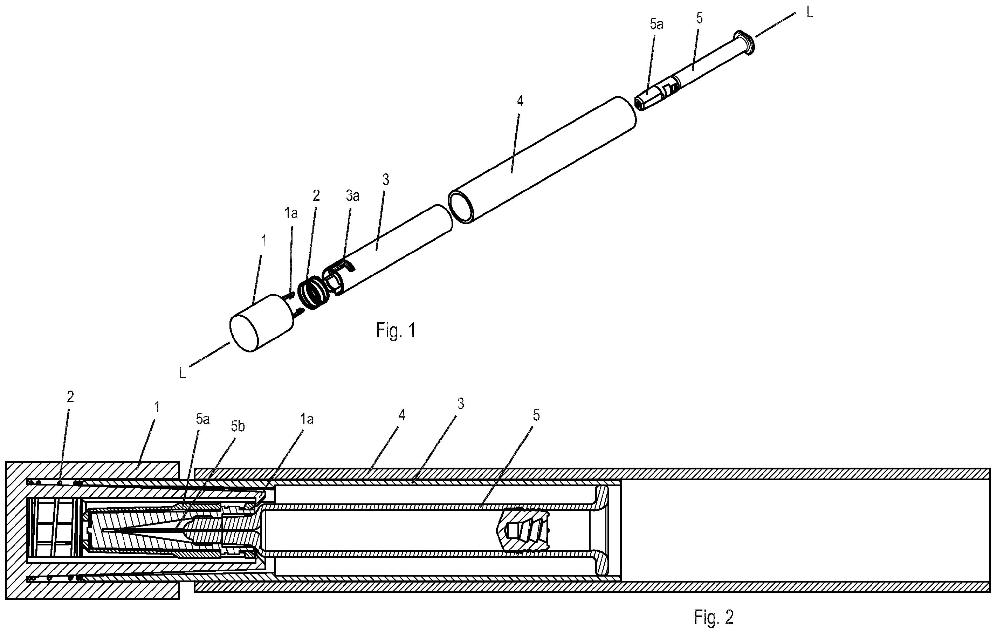

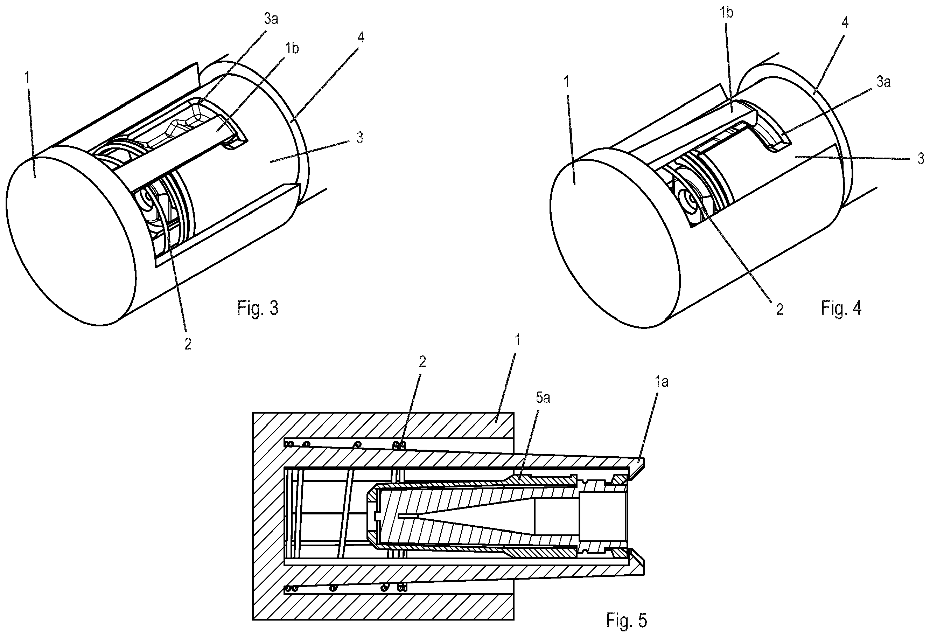

In der Figur 1 ist eine Explosionsansicht einer ersten Ausführungsform einer erfindungsgemässen Injektionsvorrichtung ersichtlich, wobei eine Kappe (1) lösbar an der Injektionsvorrichtung angeordnet ist. Die Injektionsvorrichtung kann beispielsweise in einem Auslieferungszustand die Kappe (1) an dem distalen Ende aufgesetzt haben. Die Injektionsvorrichtung umfasst ein Gehäuse (4). Das Gehäuse (4) kann als hülsenförmiges, insbesondere zylindrisches Aufnahmegehäuse mit einem distalen und einem proximalen Teil ausgebildet sein. Das Gehäuse (4) dient zur Aufnahme eines Produktbehälters (5). Der Produktbehälter (5) weist eine fest verbundene Injektionsnadel (Figur 2; 5b) auf, wobei an dem Produktbehälter (5) eine Nadelschutzkappe (5a) lösbar angeordnet ist, welche die Injektionsnadel (Figur 2; 5b) umschliesst und gegenüber der Umgebung steril abdichtet. Die Injektionsvorrichtung umfasst ferner eine Nadelschutzhülse (3). Die Nadelschutzhülse (3) kann relativ zu dem Gehäuse (4) der Injektionsvorrichtung zum Auslösen einer Produktausschüttung in die proximale Richtung verschiebbar sein. Die Nadelschutzhülse (3) ist vorzugsweise drehfest mit dem Gehäuse (4) verbunden. Nach erfolgter Produktausschüttung kann die Nadelschutzhülse (3) relativ zu dem Gehäuse (4) in die distale Richtung verschiebbar sein, um die Spitze der Injektionsnadel (Figur 2; 5b) abzudecken, um eine Verletzungsgefahr zu verringern. Die Kappe (1) umfasst ein oder mehrere Eingriffselemente (1a). Das Eingriffselement (1a) dient beim Entfernen der Kappe (1) von der Injektionsvorrichtung dazu, das Entfernen der Nadelschutzkappe (5a) von dem Produktbehälter (5) zu bewirken. Dazu ist das Eingriffselement (1a) hakenförmig ausgebildet oder weist einen oder mehrere Haken auf. Der Haken ist derart ausgebildet, dass der Haken in oder an die Nadelschutzkappe (5a) greifen kann, um in einer Eingriffsposition mit der Nadelschutzkappe (5a) zu sein oder zu gelangen. Das Eingriffselement (1a) ist vorzugsweise aus Metall, insbesondere aus Stahl, insbesondere bevorzugt aus rostfreiem Stahl oder Federstahl gebildet. Alternativ kann das Eingriffselement (1a) aus Kunststoff gebildet sein. Das Eingriffselement (1a) ist vorzugsweise axial- und drehfest mit der Kappe (1) verbunden. Die Kappe (1) ist vorzugsweise aus Kunststoff gebildet. Alternativ können die Kappe (1) und das Eingriffselement (1a) einstückig ausgebildet sein und vorzugsweise aus Metall, insbesondere aus Stahl, besonders bevorzugt aus rostfreiem Stahl oder Federstahl gebildet sein. Die Kappe (1) ist vorzugsweise über die Nadelschutzhülse (3) an dem distalen Ende des Gehäuses (4) lösbar vorgesehen. Alternativ oder zusätzlich kann die Kappe (1) direkt an dem distalen Ende des Gehäuses (4) lösbar vorgesehen sein. Die Injektionsvorrichtung umfasst ferner ein Energiespeicherelement (2) in Form einer Feder (2). Die Feder (2) ist in distale Richtung an der Kappe (1) und in die proximale Richtung an der Nadelschutzhülse (3) abgestützt. Alternativ kann die Feder (2) in die proximale Richtung an dem Gehäuse (4) abgestützt sein. Die Feder (2) ist derart in der Injektionsvorrichtung vorgespannt gelagert, dass die Nadelschutzkappe (5a) zumindest teilweisen durch eine Kraft der Feder (2) von dem Produktbehälter (5) entfernt werden kann. Die Feder (2) ist zumindest teilweise in der Kappe (1) befestigt. An der Nadelschutzhülse (3) oder alternativ an dem Gehäuse (4) der Injektionsvorrichtung ist ein Element (3a) vorgesehen, welches in Führungseingriff mit einem an der Kappe (1) vorgesehenen Gegenelement (Figur 3; 1b) ist. Das Element (3a) und/oder das Gegenelement (Figur 3; 1b) können als eine Führungskulisse/Führungsnut oder eine Führungsnocke ausgebildet sein. Die Kappe (1) ist relativ zu dem Gehäuse (4) oder der Nadelschutzhülse (3) drehbar angeordnet, um die Kappe (1) von dem Injektionsgerät zu entfernen. Das Element (3a) und das Gegenelement (Figur 3; 1b) können relativ zu einander drehbar sein. Das Element (3a) und das Gegenelement (Figur 3; 1b) sind dazu ausgelegt, in einer ersten relativen Drehposition um die Längsachsel (L) zwischen dem Element (3a) und dem Gegenelement (Figur 3; 1b) ein Entfernen der Kappe (1) von der Injektionsvorrichtung zu verhindern und in einer zweiten relativen Drehposition um die Längsachsel (L) zwischen dem Element (3a) und dem Gegenelement (Figur 3; 1b) ein Entfernen oder Abziehen der Kappe (1) von der Injektionsvorrichtung zu ermöglichen. Somit kann die Kappe (1) mit Hilfe der Kraft der Feder (2) von der Injektionsvorrichtung entfernt werden.

In der Figur 2 ist eine Längsschnittansicht der Injektionsvorrichtung gemäss Figur 1 dargestellt, wobei die Kappe (1) relativ zu der Nadelschutzhülse (3) in einer ersten relativen Drehposition angeordnet ist. Das Eingriffselement (1a) der Kappe ist in der Eingriffsposition. Das Eingriffselement (1a), insbesondere ein kurzer Schenkel des Eingriffselements (1a) greift an die Kante der Nadelschutzkappe (5a). Die Feder (2) ist in die distale Richtung an der Kappe (1) und in die proximale Richtung an der Nadelschutzhülse (3) vorgespannt abgestützt. Ferner ist die Kappe (1) über eine lösbare Verbindung, insbesondere über die Nadelschutzhülse (3) mit dem Gehäuse (4) der Injektionsvorrichtung verbunden. Ferner sind das Element (3a) der Nadelschutzhülse (3) und das Gegenelement (1b) der Kappe (1) in einem Führungseingriff. Das Element (3a) der Nadelschutzhülse (3) und das Gegenelement (1b) der Kappe (1) sind in der ersten relativen Drehposition, wie in der Figur 3 ersichtlich ist. Das Element (3a) der Nadelschutzhülse (3) und das Gegenelement (1b) der Kappe (1) sind in der ersten relativen Drehposition derart relativ zu einander angeordnet, dass ein Entfernen der Kappe (1) von der Injektionsvorrichtung verhindert wird. In diesem Ausführungsbeispiel sind das Element (3a) der Nadelschutzhülse (3) als Führungskulisse/Führungsnut, insbesondere L-förmig und das Gegenelement (1b) der Kappe (1) als Nocke ausgebildet. Andere Ausgestaltungen des Elements (3a) und des Gegenelements (1b) können vorgesehen sein. Ferner kann die Führungskulisse/Führungsnut einen Absatz oder eine Treppe umfassen. Der Absatz oder die Treppe kann dazu dienen dass das als Nocke ausgebildete Gegenelement (1a) während der Führung in dem als Führungskulisse/Führungsnut ausgebildeten Element (3a) gebremst wird.

Um die Kappe (1) von der Injektionsvorrichtung zu entfernen, dreht der Benutzer die Kappe (1) um die Längsachse (L) relativ zu dem Gehäuse (4) und/oder zu der Nadelschutzhülse (3). Dazu kann eine visuelle Markierung, insbesondere in Form eines Zeichens an der Injektionsvorrichtung vorgesehen sein, welche anzeigt, in welche Richtung eine relative Drehung der Kappe (1) zu dem Gehäuse (4) und/oder zu der Nadelschutzhülse (3) um die Längsachse (L) möglich ist. Dabei gelangt das Gegenelement (1b) der Kappe (1) relativ zu dem Element (3a) der Nadelschutzhülse (3) von der ersten relativen Drehposition zu einer zweiten relativen Drehposition. In der zweiten relativen Drehposition sind das Gegenelement (1b) der Kappe (1) und das Element (3a) der Nadelschutzhülse derart relativ zu einander angeordnet, dass ein Entfernen der Kappe (1) von der Injektionsvorrichtung möglich ist, wie in der Figur 4 dargestellt ist.

Durch eine kombinierte/serielle Axial-Dreh-Bewegung oder einer Axialbewegung der Kappe (1) zu dem Gehäuse (4) und/oder zu der Nadelschutzhülse (3) kann die Kappe (1) von der Injektionsvorrichtung entfernt werden. Dabei kann das Eingriffselement (1a) der Kappe (1) in die distale Richtung bewegt werden. Das Eingriffselement (1a) der Kappe (1) kann dabei gleichzeitig oder nachfolgend in die distale Richtung bewegt werden. Das Eingriffselement (1a) der Kappe (1) ist oder wird in Eingriff mit der Nadelschutzkappe (5a) gebracht. Besonders bevorzugt greift das Eingriffselement (1a), insbesondere der kurze Schenkel des Eingriffselements (1a) an die Kante der Nadelschutzkappe (5a). Die axiale Bewegung des Eingriffselements (1a) wird durch die Feder (2) unterstützt. Dabei entspannt sich die Feder (2) zumindest teilweise, wobei die Kraft der Feder (2) zumindest teilweise das manuelle Entfernen der Nadelschutzkappe (5a) von dem Produktbehälter (5) unterstützt.

Das Entfernen der Kappe (1) von der Injektionsvorrichtung bewirkt das Entfernen der Nadelschutzkappe (5a) von dem Produktbehälter (5). Nach dem Entfernen der Kappe (1) von der Injektionsvorrichtung verbleibt die Nadelschutzkappe (5a) in der Kappe (1). Die Nadelschutzkappe (5a) wird in der Kappe (1), insbesondere durch das Eingriffselement (1a) gehalten, wie in der Figur 5 dargestellt ist. Danach kann der Benutzer mit der Injektionsvorrichtung eine Injektion tätigen.

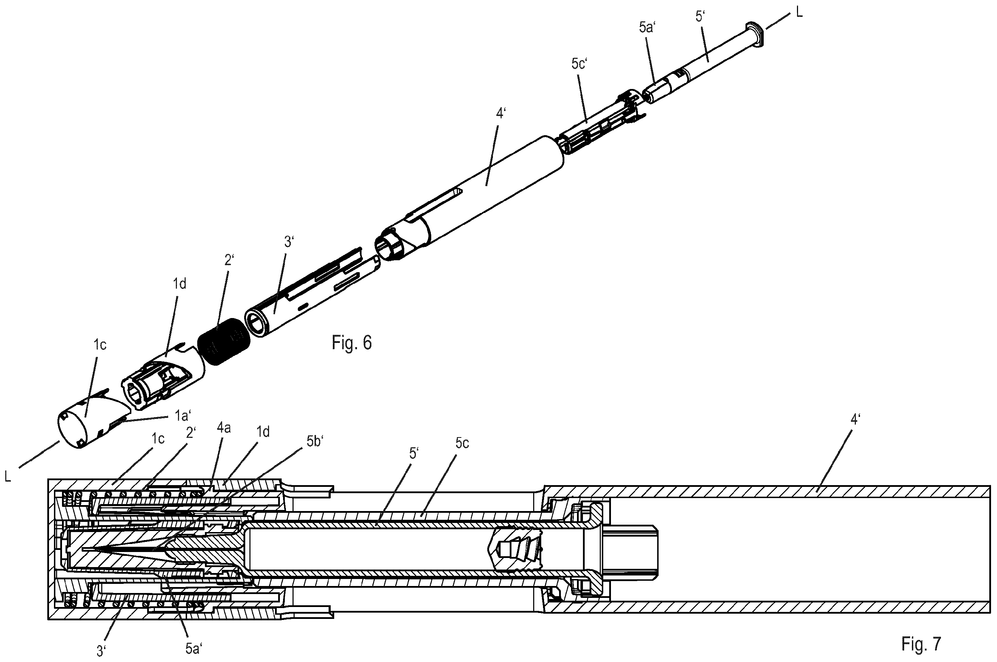

In der Figur 6 ist eine Explosionsansicht einer zweiten Ausführungsform einer erfindungsgemässen Injektionsvorrichtung dargestellt. Die Injektionsvorrichtung unterscheidet sich von der Injektionsvorrichtung der ersten Ausführungsform im Wesentlichen nur in Bezug auf die Ausgestaltung und in Bezug auf das Zusammenwirken der Kappe (1c, 1d), der Nadelschutzhülse (3'), der Feder (2') und des Gehäuses (4'). In dieser Ausführungsform ist die Kappe (1c, 1d) aus spritzgusstechnischen Gründen zweiteilig, nämlich aus einem ersten (1c) und einem zweiten Teil (1d) ausgestaltet, wobei auch eine einteilige Kappe (1c, 1d) vorgesehen werden kann.

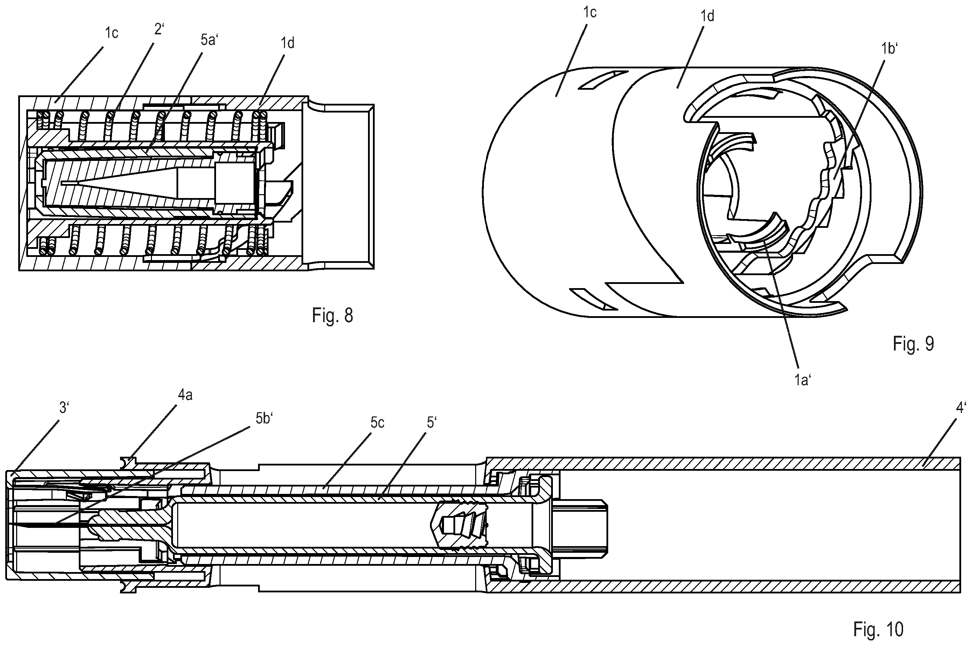

In der Figur 7 ist eine Längsschnittansicht der Injektionsvorrichtung gemäss Figur 6 dargestellt, wobei die Kappe (1c, 1d) relativ zu dem Gehäuse (4') in einer ersten relativen Drehposition angeordnet ist. Ein Eingriffselement (1a') befindet sich in der Eingriffsposition, wobei das Eingriffselement (1a') an die Kante einer an einem Produktbehälter (5') angeordneten Nadelschutzkappe (5a') greift. Die Nadelschutzkappe (5a') umgibt dabei eine Injektionsnadel (5b') des Produktbehälters (5'). Ferner ist in diesem Ausführungsbeispiel der Produktbehälter (5') axial- und drehfest in einem Produktbehälterhalter (5c) aufgenommen, welcher axial- und drehfest in dem Gehäuse (5') angeordnet ist. Die Feder (2') ist in die distale Richtung an der Kappe (1c, 1d) und in die proximale Richtung an dem Gehäuse (4') vorgespannt abgestützt. Die Feder (2') wird mit einem oder mehreren Halteelementen in der Kappe (1) gehalten. Das als Nocke ausgebildete Element (Figur 10; 4a) des Gehäuses (4') ist in Führungseingriff mit der als Führungskulisse/Führungsnut ausgebildete Gegenelement (Figur 9; 1b') der Kappe (1c, 1d).Das Element (4a) des Gehäuses (4') und das Gegenelement (Figur 9; 1b') der Kappe (1c, 1d) sind in der ersten relativen Drehposition derart relativ zu einander angeordnet, dass ein Entfernen der Kappe (1c, 1d) von der Injektionsvorrichtung verhindert wird. Die Führungskulisse des Gegenelements (Figur 9; 1b') der Kappe (1c, 1d) ist in diesem Ausführungsbeispiel treppenförmig ausgebildet. Die Treppe kann dazu dienen, dass das als Nocke ausgebildete Element (4a) des Gehäuses während der Führung in dem als Führungskulisse/Führungsnut/Treppennut ausgebildeten Gegenelement (Figur 9; 1b') der Kappe (1c, 1d) gebremst wird. Besonders bevorzugt kann das als Nocke ausgebildete Element (Figur 10; 4a) des Gehäuses (4) als Abstützelements der Feder (2') in die proximale Richtung dienen.

Um die Kappe (1c, 1d) von der Injektionsvorrichtung zu entfernen, dreht der Benutzer die Kappe (1c, 1d) um die Längsachse (L) relativ zu dem Gehäuse (4') und/oder zu der Nadelschutzhülse (3'). Dabei bewegt sich das Gegenelement (1b') der Kappe (1c, 1d) relativ zu dem Element (Figur 10; 4a) des Gehäuses (4') von der ersten relativen Drehposition zu einer zweiten relativen Drehposition. Bei dieser Bewegung gleitet das als Nocke ausgebildete Element (Figur 10; 4a) des Gehäuses (4') entlang der als Treppe ausgebildete Gegenelement (Figur 9; 1b') der Kappe (1c, 1d). Das als Treppe ausgebildete Gegenelement (Figur 9; 1b') der Kappe (1c, 1d) bremst die Bewegung der als Nocke ausgebildete Element (Figur 10; 4a) des Gehäuses (4'). In der zweiten relativen Drehposition sind das Gegenelement (1b') der Kappe (1c, 1d) und das Element (4a) des Gehäuses (4') derart relativ zu einander angeordnet, dass ein Entfernen der Kappe (1c, 1d) von der Injektionsvorrichtung möglich ist.

Durch eine kombinierte Axial-Dreh-Bewegung oder einer Axialbewegung der Kappe (1c, 1d) zu dem Gehäuse (4') und/oder zu der Nadelschutzhülse (3') kann die Kappe (1c, 1d) von der Injektionsvorrichtung entfernt werden, wobei das Eingriffselement (1a') der Kappe (1c, 1d) an die Kante der Nadelschutzkappe (5a') greift und die Nadelschutzkappe (5a') mitnimmt. Nach dem Entfernen der Nadelschutzkappe (5a') von dem Produktbehälter (5') kann der Benutzer mit der Injektionsvorrichtung eine Injektion tätigen.

### Bezugszeichen:

- 1: Kappe
- 1a, 1a': Eingriffselement
- 1b, 1b': Gegenelement
- 1c: erster Teil der Kappe
- 1d: zweiter Teil der Kappe
- 2, 2': Energiespeicherelement, Feder
- 3, 3': Nadelschutzhülse
- 3a: Element der Nadelschutzhülse
- 4, 4': Gehäuse
- 4a: Element des Gehäuses
- 5, 5': Produktbehälter
- 5a, 5a': Nadelschutzkappe
- 5b, 5b': Injektionsnadel
- 5c: Produktbehälterhalter
- L: Längsachse

## Patentansprüche

1. Injektionsvorrichtung meiner Längsachse (L) mit:
- einem Gehäuse (4; 4') zur Aufnahme eines Produktbehälters (5; 5'), wobei der Produktbehälter (5; 5') eine fest verbundene Injektionsnadel (5b; 5b') aufweist, wobei an dem Produktbehälter (5; 5') eine Nadelschutzkappe (5a; 5a') lösbar angeordnet ist, welche die Injektionsnadel (5b; 5b') umschliesst und gegenüber der Umgebung steril abdichtet,
- einer Kappe (1; 1c, 1d), welche lösbar an einem distalen Ende des Gehäuses (4; 4') vorgesehen ist, wobei die Kappe (1; 1c, 1d) ein Eingriffselement (1a; 1a') umfasst, um beim Entfernen der Kappe von der Injektionsvorrichtung das Entfernen der Nadelschutzkappe (5a; 5a') von dem Produktbehälter (5; 5') zu bewirken,
- ein Energiespeicherelement (2, 2') zum zumindest teilweisen Entfernen der Nadelschutzkappe (5a, 5a') von dem Produktbehälter (5; 5'), welches in die distale Richtung an der Kappe (1; 1c, 1d) abgestützt ist und in die proximale Richtung an dem Gehäuse (4; 4') abgestützt ist oder an einer Nadelschutzhülse (3; 3'), welche axial bewegbar an dem Gehäuse (4, 4') angeordnet ist und welche nach erfolgter Injektion distal über das distale Ende der Injektionsnadel (5b; 5b') steht, abgestützt ist, wobei
- die Kappe (1; 1c, 1d) relativ zu dem Gehäuse (4; 4') und/oder relativ zu der Nadelschutzkappe (5a; 5a') drehbar ist,
- ein Element (3a; 4a), welches an dem Gehäuse (4) oder an der Nadelschutzhülse (3) vorsehen ist und welches in Führungseingriff mit einem an der Kappe (1; 1c, 1d) vorgesehenen Gegenelement (1b; 1b') ist, wobei das Element (3a; 4a) und das Gegenelement (1b; 1b') dazu ausgelegt sind, in einer ersten relativen Drehposition um die Längsachse (L) zwischen dem Element (3a; 4a) und dem Gegenelement (1b; 1b') ein Entfernen der Kappe (1; 1c, 1d) von der Injektionsvorrichtung zu verhindern und in einer zweiten relativen Drehposition um die Längsachse (L) zwischen dem Element (3a; 4a) und dem Gegenelement (1b; 1b') ein Entfernen der Kappe (1; 1c, 1d) von der Injektionsvorrichtung zu ermöglichen,
**dadurch gekennzeichnet, dass**
das Element (3a; 4a) oder das Gehäuse (4) ein Rückdrehsicherungselement und das Gegenelement (1b; 1b') oder die Kappe (1; 1c, 1d) ein Rückdrehsicherungsgegenelement aufweisen, um eine Drehung der Kappe (1; 1c, 1d) um die Längsachse (L) in eine Richtung zu ermöglichen und in Gegenrichtung zu sperren.

2. Injektionsvorrichtung nach Anspruch 1, **dadurch gekennzeichnet, dass** das Element (3a; 4a) und/oder das Gegenelement (1b; 1b') als eine Führungskulisse/Führungsnut oder eine Führungsnocke ausgebildet ist.

3. Injektionsvorrichtung nach Anspruch 2, **dadurch gekennzeichnet, dass** die Führungskulisse/Führungsnut ein Gewinde umfasst oder gewindeförmig ausgebildet ist.

4. Injektionsvorrichtung nach Anspruch 2 oder 3, **dadurch gekennzeichnet, dass** die Führungskulisse/Führungsnut einen Absatz oder eine Treppe umfasst oder treppenförmig oder L-förmig ausgebildet ist.

5. Injektionsvorrichtung nach einem der vorhergehenden Ansprüchen, **dadurch gekennzeichnet, dass** das Energiespeicherelement (2; 2') als Feder (2; 2') oder als Magnet ausgebildet ist.

6. Injektionsvorrichtung nach einem der vorhergehenden Ansprüche, **dadurch gekennzeichnet, dass** das Element (4a) oder das Gegenelement als Abstützelement des Energiespeicherelements (2') in die proximale Richtung dient.

7. Injektionsvorrichtung nach einem der vorhergehenden Ansprüche, **dadurch gekennzeichnet, dass** das Eingriffselement (1a; 1a') axial- und drehfest mit der Kappe (1; 1c, 1d) verbunden ist.

8. Injektionsvorrichtung nach einem der vorhergehenden Ansprüche, **dadurch gekennzeichnet, dass** das Element (3a; 4a) axial- und drehfest mit dem Gehäuse (4; 4') oder der Nadelschutzhülse (3, 3') verbunden ist und das Gegenelement (1b; 1b') axial- und drehfest mit der Kappe (1; 1c, 1d) verbunden ist.

9. Injektionsvorrichtung nach einem der vorhergehenden Ansprüche, **dadurch gekennzeichnet, dass** die Injektionsvorrichtung eine visuelle Markierung, insbesondere in Form eines Zeichens aufweist, um anzuzeigen, in welche Richtung eine relative Drehung der Kappe (1; 1c, 1d) zu dem Gehäuse (4; 4') oder zu der Nadelschutzhülse (3; 3') um die Längsachse (L) möglich ist.

10. Injektionsvorrichtung nach einem der vorhergehenden Ansprüche, **dadurch gekennzeichnet, dass** das Eingriffselement (1b; 1b') einen oder mehrere Haken aufweist oder hakenförmig ausgebildet ist.

11. Injektionsvorrichtung nach einem der vorhergehenden Ansprüche, **dadurch gekennzeichnet, dass** das Eingriffselement (1b; 1b') elastisch oder plastisch verformbar ist.

12. Injektionsvorrichtung nach einem der vorhergehenden Ansprüche, **dadurch gekennzeichnet, dass** eine Nadelschutzhülse (3; 3') vorgesehen ist, wobei die Kappe (1; 1c, 1d) über die Nadelschutzhülse (3; 3') mit dem Gehäuse (4; 4') lösbar vorgesehen ist.

## Claims

1. Injection device of my longitudinal axis (L), comprising:
- a housing (4; 4') for receiving a product container (5; 5'), the product container (5; 5') having a fixedly connected injection needle (5b; 5b'), a needle protection cap (5a; 5a') being detachably arranged on the product container (5; 5'), which needle protection cap encloses the injection needle (5b; 5b') and seals the injection needle from the surroundings in a sterile manner,
- a cap (1; 1c, 1d) detachably provided at a distal end of the housing (4; 4'), the cap (1; 1c, 1d) comprising an engagement element (1a; 1a') to remove the needle protection cap (5a; 5a') from the product container (5; 5') when the cap is removed from the injection device,
- an energy storage element (2, 2') for at least partially removing the needle protection cap (5a, 5a') from the product container (5; 5'), which energy storage element is supported in the distal direction on the cap (1; 1c, 1d) and in the proximal direction on the housing (4; 4') or is supported on a needle protection sleeve (3; 3') axially movably arranged on the housing (4, 4') and projecting distally over the distal end of the injection needle (5b; 5b') after injection,
- the cap (1; 1c, 1d) being rotatable relative to the housing (4; 4') and/or relative to the needle protection cap (5a; 5a'),
- an element (3a; 4a) which is provided on the housing (4) or on the needle protection sleeve (3) and guidingly engages with a counter element (1b; 1b') provided on the cap (1; 1c, 1d), the element (3a; 4a) and the counter element (1b; 1b') being designed to prevent the cap (1; 1c, 1d) from being removed from the injection device in a first relative rotational position about the longitudinal axis (L) between the element (3a; 4a) and the counter element (1b; 1b') and to allow the cap (1; 1c, 1d) to be removed from the injection device in a second relative rotational position about the longitudinal axis (L) between the element (3a; 4a) and the counter element (1b; 1b'),
**characterized in that**
the element (3a; 4a) or the housing (4) has an anti-reverse-rotation element and the counter element (1b; 1b') or the cap (1; 1c, 1d) has an anti-reverse-rotation counter element, to allow rotation of the cap (1; 1c, 1d) about the longitudinal axis (L) in one direction and to block it in the opposite direction.

2. Injection device according to claim 1, **characterized in that** the element (3a; 4a) and/or the counter element (1b; 1b') is in the form of a guide slot/guide groove or a guide cam.

3. Injection device according to claim 2, **characterized in that** the guide slot/guide groove comprises a thread or is thread-shaped.

4. Injection device according to claim 2 or 3, **characterized in that** the guide slot/guide groove comprises a shoulder or a step or is stepped or L-shaped.

5. Injection device according to any of the preceding claims,
**characterized in that** the energy storage element (2; 2') is in the form of a spring (2; 2') or a magnet.

6. Injection device according to any of the preceding claims,
**characterized in that** the element (4a) or the counter element serves as a support element for the energy storage element (2') in the proximal direction.

7. Injection device according to any of the preceding claims,
**characterized in that** the engagement element (1a; 1a') is connected to the cap (1; 1c, 1d) in an axially fixed manner for conjoint rotation.

8. Injection device according to any of the preceding claims,
**characterized in that** the element (3a; 4a) is connected to the housing (4; 4') or the needle protection sleeve (3, 3') in an axially fixed manner for conjoint rotation and the counter element (1b; 1b') is connected to the cap (1; 1c, 1d) in an axially fixed manner for conjoint rotation.

9. Injection device according to any of the preceding claims,
**characterized in that** the injection device has a visual marking, in particular in the form of a symbol, to indicate in which direction a relative rotation of the cap (1; 1c, 1d) with respect to the housing (4; 4') or to the needle protection sleeve (3; 3') about the longitudinal axis (L) is possible.

10. Injection device according to any of the preceding claims,
**characterized in that** the engagement element (1b; 1b') has one or more hooks or is hook-shaped.

11. Injection device according to any of the preceding claims,
**characterized in that** the engagement element (1b; 1b') is elastically or plastically deformable.

12. Injection device according to any of the preceding claims,
**characterized in that** a needle protection sleeve (3; 3') is provided, the cap (1; 1c, 1d) being detachably provided with the housing (4; 4') via the needle protection sleeve (3; 3').

## Revendications

1. Dispositif d'injection de mon axe longitudinal (L) comportant :
- un boîtier (4 ; 4') permettant de recevoir un récipient pour produit (5 ; 5'), dans lequel le récipient pour produit (5 ; 5') présente une aiguille d'injection (5b ; 5b') reliée de manière fixe, dans lequel un capuchon de protection d'aiguille (5a ; 5a') est disposé de manière amovible sur le récipient pour produit (5 ; 5'), lequel capuchon entoure l'aiguille d'injection (5b ; 5b') et rend celle-ci étanche de manière stérile vis-à-vis de l'environnement,
- un capuchon (1 ; 1c, 1d) prévu de manière amovible à une extrémité distale du boîtier (4 ; 4'), dans lequel le capuchon (1 ; 1c, 1d) comprend un élément de mise en prise (1a ; 1a') pour, lorsque le capuchon est retiré du dispositif d'injection, permettre le retrait du capuchon de protection d'aiguille (5a ; 5a') du récipient pour produit (5 ; 5'), -un élément d'accumulation d'énergie (2, 2') pour le retrait au moins partiel du capuchon de protection d'aiguille (5a, 5a') du récipient pour produit (5 ; 5'), lequel élément s'appuie sur le capuchon (1 ; 1c, 1d) dans la direction distale et s'appuie sur le boîtier (4 ; 4') dans la direction proximale ou s'appuie sur un manchon de protection d'aiguille (3 ; 3') qui est disposé de manière à être axialement mobile sur le boîtier (4, 4') et qui, une fois l'injection effectuée, se trouve distalement au-dessus de l'extrémité distale de l'aiguille d'injection (5b ; 5b'), dans lequel
- le capuchon (1 ; 1c, 1d) peut tourner par rapport au boîtier (4 ; 4') et/ou par rapport au capuchon de protection d'aiguille (5a ; 5a'),
- un élément (3a ; 4a) qui est prévu sur le boîtier (4) ou sur le manchon de protection d'aiguille (3) et qui est en prise par guidage avec un contre-élément (1b ; 1b') prévu sur le capuchon (1 ; 1c, 1d), dans lequel l'élément (3a ; 4a) et le contre-élément (1b ; 1b') sont conçus pour empêcher un retrait du capuchon (1 ; 1c, 1d) du dispositif d'injection dans une première position de rotation relative autour de l'axe longitudinal (L) entre l'élément (3a ; 4a) et le contre-élément (1b ; 1b') et pour permettre un retrait du capuchon (1 ; 1c, 1d) du dispositif d'injection dans une seconde position de rotation relative autour de l'axe longitudinal (L) entre l'élément (3a ; 4a) et le contre-élément (1b ; 1b'),
**caractérisé en ce que**
l'élément (3a ; 4a) ou le boîtier (4) présente un élément antirotation en sens inverse et le contre-élément (1b ; 1b') ou le capuchon (1 ; 1c, 1d) présente un contre-élément antirotation en sens inverse pour permettre une rotation du capuchon (1 ; 1c, 1d) autour de l'axe longitudinal (L) dans un sens et pour le bloquer dans le sens inverse.

2. Dispositif d'injection selon la revendication 1, **caractérisé en ce que** l'élément (3a ; 4a) et/ou le contre-élément (1b ; 1b') est réalisé en tant que coulisse de guidage/rainure de guidage ou en tant que came de guidage.

3. Dispositif d'injection selon la revendication 2, **caractérisé en ce que** la coulisse de guidage/rainure de guidage comprend un filetage ou est réalisée sous forme de filetage.

4. Dispositif d'injection selon la revendication 2 ou 3, **caractérisé en ce que** la coulisse de guidage/rainure de guidage comprend un épaulement ou un escalier ou est réalisée en forme d'escalier ou en forme de L.

5. Dispositif d'injection selon l'une des revendications précédentes,
**caractérisé en ce que** l'élément d'accumulation d'énergie (2 ; 2') est réalisé en tant que ressort (2 ; 2') ou en tant qu'aimant.

6. Dispositif d'injection selon l'une des revendications précédentes,
**caractérisé en ce que** l'élément (4a) ou le contre-élément sert d'élément d'appui de l'élément d'accumulation d'énergie (2') dans la direction proximale.

7. Dispositif d'injection selon l'une des revendications précédentes,
**caractérisé en ce que** l'élément de mise en prise (1a ; 1a') est relié de manière fixe axialement et en rotation au capuchon (1 ; 1c, 1d).

8. Dispositif d'injection selon l'une des revendications précédentes,
**caractérisé en ce que** l'élément (3a ; 4a) est relié de manière fixe axialement et en rotation au boîtier (4 ; 4') ou au manchon de protection d'aiguille (3, 3') et le contre-élément (1b ; 1b') est relié de manière fixe axialement et en rotation au capuchon (1 ; 1c, 1d).

9. Dispositif d'injection selon l'une des revendications précédentes,
**caractérisé en ce que** le dispositif d'injection présente un repère visuel, en particulier sous la forme d'un marquage, pour indiquer dans quel sens une rotation relative du capuchon (1 ; 1c, 1d) par rapport au boîtier (4 ; 4') ou par rapport au manchon de protection d'aiguille (3 ; 3') est possible autour de l'axe longitudinal (L).

10. Dispositif d'injection selon l'une des revendications précédentes,
**caractérisé en ce que** l'élément de mise en prise (1b ; 1b') présente un ou plusieurs crochets ou est réalisé en forme de crochet.

11. Dispositif d'injection selon l'une des revendications précédentes,
**caractérisé en ce que** l'élément de mise en prise (1b ; 1b') est déformable élastiquement ou plastiquement.

12. Dispositif d'injection selon l'une des revendications précédentes,
**caractérisé en ce qu'**un manchon de protection d'aiguille (3 ; 3') est prévu, dans lequel le capuchon (1 ; 1c, 1d) est prévu de manière amovible avec le boîtier (4 ; 4') par l'intermédiaire du manchon de protection d'aiguille (3 ; 3').
